# EUROPEAN PATENT APPLICATION

(11) **EP 4 790 135 A1**
(43) Date of publication of application: **12.08.2026**
(21) Application number: 25305171.8
(22) Date of filing: 05.02.2025
(51) Int. Cl.: C12N 5/079

(54) **RETINAL ORGANOIDS**

(71) Applicant: Université de Strasbourg, 67000 Strasbourg (FR); Centre National de la Recherche Scientifique, 75016 Paris (FR); Institut National de la Santé et de la Recherche Médicale, 75013 Paris (FR)
(72) Inventor: GOLZIO, Christelle, 67400 ILLKIRCH (FR); LEMEE, Marianne, 67100 STRASBOURG (FR); LOVIGLIO, Maria Nicla, 67400 ILLKIRCH (FR)
(74) Representative: IPAZ

(57) **Abstract**

The invention relates to a human pluripotent stem cell-derived, *in vitro* generated, retinal tissue characterized in that CHD1L activity is decreased. The invention also relates to methods for obtaining the retinal tissue and methods using the same.

## Description

The present invention relates to methods for making *in vitro* retinal cultures, tissue, or retinal organoids, from pluripotent cells. It also relates to retinal organoids that replicate *in vitro* many characteristics of the retina (e.g., human or mammalian), and methods of using this retinal organoid to study disease, to identify therapeutic agents for the treatment of retinal diseases and disorders and for the treatment of retinal diseases and disorders.

### BACKGROUND

Currently, there are no treatments capable of replacing photoreceptor cells. Retinal degeneration is described as the destruction or deterioration of the retina caused by progressive and eventual death of the retinal cells. The retina is the sensory membrane or tissue that lines the inner surface of the back of the eyeball. It's composed of several layers, including one that contains specialized cells called photoreceptors. Retinal diseases vary widely, but most of them cause visual symptoms.

The disorders associated with retinal degeneration are collectively termed as retinal degenerative disorders and include prevalent retinal degenerative diseases such as diabetic retinopathy, retinopathy of prematurity, macular degeneration, Usher syndrome, Stargardt disease and Retinitis Pigmentosa.

In order to develop efficient therapies, it is vital that researchers have access to suitable model systems to test developing compounds and to study different diseases such as age-related macular degeneration (AMD) and inherited retinal dystrophies (IRDS).

Organoids are self-organising, 3D cell cultures which are valuable in many applications such as drug screening, toxicity, disease modelling, and potentially in regenerative medicine. Organoid cultures have been described modelling different organs and tissues such as retina, brain and kidney.

Organoids can be derived from isolated primary progenitor cells or pluripotent stem cells which are directed towards differentiation pathways to yield the desired cell types.

Human pluripotent stem cells, which include both human embryonic stem cells (hESCs) and human induced pluripotent stem cells (hiPSCs), hold the potential to differentiate into any cell type. As such, they can serve as comprehensive model systems of human cell genesis, particularly at early developmental stages that would otherwise be inaccessible to investigation. In addition, patient-derived hiPSC lines have a unique capacity to model human disease, although the scope of disorders amenable to this form of study is limited. Major considerations when creating hiPSC disease models include the capacity to efficiently generate, identify and isolate relevant cell populations, as well as recapitulate and assay critical aspects of the disease mechanism.

Retinal cell types are particularly well-suited for the investigation of cell development and dysfunction using pluripotent stem cell technology. The vertebrate retina harbours a modest repertoire of major cell classes sequentially produced *via* a conserved series of events. Furthermore, the effects of inherited and acquired retinal degenerative diseases (RDD) are often limited initially to a specific cell class, which simplifies the study of cellular mechanisms that incite RDD and the evaluation of potential therapies.

Previous studies have demonstrated the ability of human pluripotent stem cells to differentiate along the retinal lineage with varying efficiencies, with one protocol achieving a near uniform retinal cell fate using the WA01 hESC line (Lamba et al., 2011). However, pluripotent stem cell-derived retinal cells, particularly those from hiPSCs, are most often found in mixed populations that include some non-retinal or unidentified cell types.

Therefore, there is still a need for developing retinal tissues and especially retinal organoids obtainable with an easy process but still mimicking closely natural retinal tissue .

### DESCRIPTION

Accordingly, the invention relates a human pluripotent stem cell-derived, *in vitro* generated, retinal tissue characterized in that CHD1L activity is decreased.

The inventors unexpectedly discovered that diminishing, even inactivating, the activity of CHD1L into human pluripotent stem cells allows development of retinal tissue. In particular, the inventors discovered that diminishing, even inactivating, the activity of CHD1L gene into human pluripotent stem cells cultured with neuronal fate factors unexpectedly allows development of retinal organoid mimicking closely natural retinal organoid. Because the method for obtaining the tissue of the invention is simple, it is greatly reproductible allowing obtention of the retinal tissue of the invention is greatly reproductible. Such a breakthrough gives a new easy way for obtaining retinal tissues simplifying identification of new therapeutic agents and cure of diseases and disorders of retinal tissues in a subject.

In one embodiment, the retinal tissue is a three-dimensional retinal organoid.

In one embodiment, the human pluripotent stem cell is a human embryonic stem cell or a human induced pluripotent stem cell.

In one embodiment, the proportion of cells of the retinal tissue positives for FOXG1 is less than 1% and/or the proportion of cells positives for SIX3 is more than 40%.

In one embodiment, the retinal tissue comprises at least one group of retinal cells selected in the group consisting of retinal progenitors cells, cycling retinal progenitor cells, retinal ganglionic cells, amacrine/horizontal cells, photoreceptors/cones, Rods photoreceptors, bipolar cells and glia müller-type cells.

The invention also relates to a retinal cell line derived from the retinal tissue according as above defined.

Particularly, the retinal cell line is selected in the group consisting of a retinal progenitor cell line, a cycling retinal progenitor cell line, a retinal ganglionic cell line, an amacrine/horizontal cell line, a photoreceptors/cones cell line, Rods photoreceptors, bipolar cells and glia müller-type cells.

The invention also relates to a method for obtaining a retinal tissue as above defined comprising culturing human pluripotent stem cell wherein CHD1L activity is decreased to form a retinal tissue.

In particular, the method comprises the following steps:
a) culturing the human pluripotent stem cells wherein CHD1L activity is decreased in an inducing neuronal differentiation culture medium until obtaining an embryoid body,
b) recovering the embryoid body and placing it into a 3D embedding system,
c) culturing the embryoid body within the 3D embedding system until obtaining a retinal tissue.

The invention also relates to a method for obtaining a retinal cell line by culturing retinal progenitors cells isolated from the retinal tissue as above defined.

The invention also relates to a method for assessing the efficiency of a drug candidate for treating a retinal disease or retinal disorder comprising:
a) producing a retinal tissue with the method as above defined or a retinal cell line with the method as above defined, optionally wherein the retinal tissue or the retinal cell line has a genetic mutation or other impairment,
b) administering the drug candidate to the retinal tissue or the retinal cell line, and
c) determining the effect of the drug candidate onto the retinal tissue or the retinal cell line.

The invention also relates to the retinal tissue as above defined or the retinal cell line as above defined for use in the treatment of a retinal disease or a retinal disorder in a subject.

Particularly, the retinal disease is selected in the group consisting of inherited retinal dystrophy, diabetic retinopathy, retinopathy of prematurity, macular degeneration, age-related macular degeneration, Usher syndrome, Stargardt disease, Retinitis Pigmentosan, Bardet-Biedl syndrome, Senior-Loken syndrome, Leber congenital amaurosis, Joubert syndrome and Meckel syndrome.

In particular, the treatment is cell replacement therapy.

The invention finally relates to a pharmaceutical composition for treating or slowing the progression of a retinal degenerative disease or disorder comprising retinal cells isolated from the retinal tissue as above defined or from the retinal cell line as above defined and a pharmaceutically acceptable carrier.

### DETAILED DESCRIPTION

### Retinal tissue and organoid

In a first aspect, the invention relates to a human pluripotent stem cell-derived, *in vitro* generated, retinal tissue characterized in that CHD1L activity is decreased.

The CHD1L gene, also known as ALC1 (chromatin domain helicase/ATPase DNA binding 1-like protein gene), is located in chr1q21.1. CHD1L regulates chromosome integrity maintenance, DNA repair and transcriptional regulation by binding to DNA. Especially, the CHD1L gene has a nucleic acid sequence set forth in SEQ ID NO: 1.

As used herein, the term "decreased" regarding activity of a protein refers to an activity diminution compared to the protein activity in a comparative wild type cell by at least 50%, preferably by at least 60%, especially by at least 70%, in particular by at least 80%, particularly by at least 90%, notably by at least 95%, for example by 100%.

For diminishing the activity of CHD1L, CHD1L gene may be knocked out or knocked down and/or CHD1L protein's activity may be impaired.

As used herein, the term "knock-out," refers to the disruption of a gene that results in partial or complete suppression of the expression of at least a portion of a protein encoded by that gene and/or a reduction or elimination of activity of the polypeptide encoded by that gene, e.g., as compared to the same cell without the "knockout" (disruption). The knockout is typically the result of genomic disruptions, including transposons, tilling, homologous recombination, antisense constructs, sense constructs, or targeted disruption of the gene using, for example, a zinc finger protein, TALEN, or a CRISPR/Cas construct. The expression of the gene that is knocked-out in a cell or a tissue can be reduced by at least 80%, at least 85%, at least 90%, at least 95%, at least 98%, at least 99%, or 100%, compared to its normal expression level in the cell or tissue. In some embodiments, the expression level of a gene that is knocked-out in a cell is non-detectable using ordinary means known in the art for gene expression detection.

The phrases "disruption of the gene" and "gene disruption" refer to the deletion or insertion of a nucleic acid sequence into one region of the native DNA sequence and/or the promoter region of a gene so as to decrease or prevent expression of that gene or to decrease or eliminate activity of a protein expressed by that gene in the cell as compared to the wild-type or naturally occurring sequence of the gene.

As used herein, the term "knock-down", refers to decreasing the amount of transcription or inhibiting the translation of a particular gene by transformation. In other words, it refers to those that target gene function. In certain embodiments it is based on the RNA interference (RNAi) pathway by allowing the degradation of mRNA. Here, antisense RNA like miRNA, siRNA, and shRNA play a key role by binding to the target mRNA. The resultant RNA duplexes are degraded by the action of Dicer and RISC. In certain embodiment, it is based on antisense oligonucleotides (ASO) mechanism. ASOs are single-stranded nucleic acids that can be used to target mRNA derived from a gene of interest. ASOs can alter gene expression *via* a number of mechanisms including direct steric blockage of mRNA and ribonuclease H (RNase H) mediated degradation of mRNA. In certain embodiments, however, a knockdown can readily be accomplished using a CRISPR/Cas system, e.g., to target the transcribed RNA.

As used herein, the term "activity impairment" refers to decreasing the activity of a certain protein. In certain embodiment, it is based on a substance that inhibits or interferes with the function of the protein, for example by specifically binding to the active site of the protein or causing to the protein a structure modification. The substance may be a small molecule or an antibody. In the case of inhibition/interference of CH1DL protein function, the substance may be EIS-12656, EIS-10700 or EIS-10700.

As used herein, the term "stem cell" refers to cells that are undifferentiated or partially differentiated cells that can differentiate into various types of cells and proliferate indefinitely to produce more of the same stem cell. They are the earliest type of cell in a cell lineage. Stem cells are found in both embryonic and adult organisms, but may have slightly different properties in each. They are usually distinguished from retinal progenitor cells, which cannot divide indefinitely, and precursor or blast cells, which are usually committed to differentiating into one cell type.

As used herein, the term "pluripotent" stem cell refers to a cell that is not capable of growing into an entire organism, but is capable of giving rise to cell types originating from all three germ layers, *i.e.,* mesoderm, endoderm, and ectoderm, and may be capable of giving rise to all cell types of an organism. Pluripotency can be a feature of the cell per see, e.g. in certain stem cells, or it can be induced artificially. Examples of pluripotent stem cells include, but are not limited to, embryonic stem cells (ESC), embryonic stem cells derived from a cloned embryo obtained by nuclear transplantation (ntES), spermatogonial stem cells ("GS cells"), embryonic germ cells ("EG cells"), induced pluripotent stem cells (iPSC) and multipotent cells derived from cultured fibroblasts. In some embodiments, ESC and/or iPSC such as human ESC and/or iPSC are used in the methods and compositions disclosed herein.

In an embodiment of the invention, the human pluripotent stem cell is a human embryonic stem cell or a human induced pluripotent stem cell.

As used herein, the terms "embryonic stem cell" refer to cells that are totipotent and derived from tissue formed after fertilization but before the end of gestation, including pre-embryonic tissue (such as, for example, a blastocyst), embryonic tissue, or foetal tissue taken any time during gestation, typically but not necessarily before approximately 10-12 weeks gestation. These cells express Oct-4, SSEA-3, SSEA-4, TRA-1-60 andTRA-1-81, and appear as compact colonies having a high nucleus to cytoplasm ratio and prominent nucleolus. ESCs are commercially available from sources such as WiCell Research Institute (Madison, Wis.). Embryonic stem cells can also be obtained directly from suitable tissue, including, but not limited to human tissue, or from established embryonic cell lines. In one embodiment, embryonic stem cells are obtained as described by Thomson et al (U.S. Pat. No. 5,843,780).

As used herein, the terms "induced pluripotent stem cell" refer to pluripotent cells derived from differentiated cells. For example, iPSCs can be obtained by overexpression of transcription factors such as Oct4, Sox2, c-Myc and Klf4 according to the methods described in Takahashi et al. (Cell, 126: 663-676, 2006). Other methods for producing iPSCs are described, for example, in Takahashi et al. Cell, 131 : 861-872, 2007. Induced pluripotent stem cells exhibit morphological properties (e.g., round shape, large nucleoli and scant cytoplasm) and growth properties (e.g., doubling time of about seventeen to eighteen hours) akin to ESCs. In addition, iPS cells express pluripotent cell-specific markers (e.g., Oct-4, SSEA-3, SSEA-4, Tra-1- 60 or Tra-1-81, but not SSEA-1). Induced pluripotent stem cells, however, are not immediately derived from embryos. As used herein, "not immediately derived from embryos" means that the starting cell type for producing iPS cells is a non-pluripotent cell, such as a multipotent cell or terminally differentiated cell, such as somatic cells obtained from a post-natal individual.

As used herein, the term "tissue" refers to a structure of a cell population having a structure in which one or more types of cells having different shapes and properties are three-dimensionally arranged in a certain pattern.

As used herein, the term "retinal tissue" refers to retinal cells such as photoreceptor cells, horizontal cells, bipolar cells, amacrine cells, retinal ganglion cells, retinal pigment epithelial cells, their retinal progenitor cells, or retinal progenitor cells that constitute each retinal layer in the living retina. In one embodiment, one or more of the retinal cells described above may form a single layer or multiple layers in a certain pattern. The layers that can be included in the retinal tissue are the retinal pigment epithelial layer, the outer limiting membrane, the photoreceptor layer (outer nuclear layer), the outer plexiform layer, the inner nuclear layer, the inner plexiform layer, the ganglion cell layer, the nerve fibre layer, and the inner border. It is also called a membrane. A retinal sheet cut out from a three-dimensional retina derived from iPS cells is also a type of retinal tissue. The tissue including the retinal sheet is composed of a plurality of cells as described above, and has a pale white colour as a whole. Although the pale white tissue is not completely transparent, it is not easy to visually recognize it due to its pale colour and small volume of 0.01 to 5 mm³.

In one embodiment of the invention, the retinal tissue is a three-dimensional retinal organoid.

The term "organoid" refers to an organised mass of cell types, generated *in vitro,* that mimics at least to some degree the structure, marker expression, or function of a naturally occurring organ. The term "retinal organoid" refers to organoids which mimic human retinogenesis through formation of organized layered retinal structures that display markers for typical retinal cell types.

In one embodiment of the invention, the proportion of cells of the retinal tissue positives for FOXG1 is less than 3% and/or the proportion of cells positives for SIX3 is more than 30%.

Especially, the retinal tissue comprises at 60 days of development, a proportion of cells positives for FOXG1 is less than 3% and/or the proportion of cells positives for SIX3 is more than 30%.

The few proportion of cells positives for FOXG1 in the retinal tissue of the invention indicates the low presence of telencephalic cells within the retinal tissue. On the contrary, the high proportion of cells positives for SIX3 in the retinal tissue of the invention indicates the high presence of retinal cells within the retinal tissue. This demonstrate that unexpectedly, despite the neuronal fate factors used to produce the retinal tissue of the invention, very few of the cells, especially at 60 days of development, shows specific neuronal marker, and on the contrary large proportion of the cell shows retinal marker.

Taken together, these features indicates that the human pluripotent stem cell wherein CHD1L is knock down or knockout is directed to a retinal tissue with high proportion of cells with retinal features, and particularly, with high proportion of retinal progenitors.

In particular, the proportion of cells of the retinal tissue positives for FOXG1 is less than 2%, especially less than 1%, preferably less than 0.8 %, particularly less than 0.6 %, notably less than 0.5%, especially less than 0.4%, preferably less than 0.3%, for example 0.2%.

In particular, the proportion of cells of the retinal tissue positives for SIX3 is more than 35%, especially more than 40%, preferably more than 42%, notably more than 45%, especially more than 47%, preferably more than 48%, for example 49%.

In one embodiment of the invention, at least a part of the cells of the retinal tissue are further positives for at least one retinal fate specification gene selected in the group consisting of OTX2, VSX2 and RORB.

In one embodiment of the invention, the retinal tissue comprises at least one group of retinal cells selected in the group consisting of retinal progenitors cells, cycling retinal progenitor cells, retinal ganglionic cells, amacrine/horizontal cells, photoreceptors/cones, Rods photoreceptors, bipolar cells and glia müller-type cells.

With preference, the retinal tissue comprises at least one group of retinal cells selected in the group consisting of retinal progenitors cells, cycling retinal progenitor cells, retinal ganglionic cells, amacrine/horizontal cells and photoreceptors/cones.

With preference, the retinal tissue comprises at 60 days of development at least 50 cells of at least one group of retinal cells selected in the group consisting of retinal progenitors cells, cycling retinal progenitor cells, retinal ganglionic cells, amacrine/horizontal cells and photoreceptors/cones.

Further, with preference, the retinal tissue comprises at 60 days of development at least 100 cells of at least one group of retinal cells selected in the group consisting of retinal progenitors cells, retinal ganglionic cells, amacrine/horizontal cells and photoreceptors/cones.

Further, with preference, the retinal tissue comprises at 60 days of development at least 200 cells of at least one group of retinal cells selected in the group consisting of retinal progenitors cells, retinal ganglionic cells and photoreceptors/cones.

With preference, the retinal tissue comprises at 60 days of development at least 300 cells of at least one group of retinal cells selected in the group consisting of retinal progenitors cells, retinal ganglionic cells and photoreceptors/cones.

In one embodiment of the invention, the retinal tissue comprises at 60 days of development less than ten cells of at least one group of cells selected in the group consisting of cycling radial glial cells, excitatory neurons, immature neurons and radial glial cells.

With preference, the retinal tissue comprises at 60 days of development less than five cells of at least one group of cells selected in the group consisting of cycling radial glial cells, excitatory neurons, immature neurons and radial glial cells.

With preference, the retinal tissue does not comprise at 60 days of development at least one group of cells selected in the group consisting of cycling radial glial cells and excitatory neurons.

### Retinal cell line

The invention also relates to a retinal cell line derived from the retinal tissue as above defined.

Because the retinal tissue of the invention advantageously comprises retinal progenitors and especially a high proportion of retinal progenitors at 60 days of development, this make it possible to isolate and culture the latter to obtain therefrom different retinal cell lines.

In particular, the retinal cell line of the invention is selected in the group consisting of a retinal progenitor cell line, a cycling retinal progenitor cell line, a retinal ganglionic cell line, an amacrine/horizontal cell line and a photoreceptors/cones cell line, Rods photoreceptors, bipolar cells and glia müller-type cells.

### Methods of production

The invention also relates to a method for obtaining a retinal tissue as above defined comprising culturing human pluripotent stem cell wherein CHD1L activity is decreased to form a retinal tissue

Especially, the human pluripotent stem cell wherein CHD1L activity is decreased are cultured under conditions for allowing the cells to differentiate into neuronal cells. Unexpectedly, those conditions does not give a neuronal fate to the human pluripotent stem cell wherein CHD1L activity is decreased but on the contrary allows forming retinal tissue.

In one embodiment, the human pluripotent stem cell are cultured during at least 30 days, especially at least 60 days, notably at least 100 days, in particular at least 150 days, for example 200 days.

The method of the invention advantageously does not require a checkerboard scraping step.

In one embodiment, the method comprises the following steps:
a) culturing the human pluripotent stem cells wherein CHD1L activity is decreased in an inducing neuronal differentiation culture medium until obtaining an embryoid body,
b) recovering the embryoid body and placing it into a 3D embedding system,
c) culturing the embryoid body within the 3D embedding system until obtaining a retinal tissue.

As used herein, the term "embryoid body" refers to three-dimensional aggregates. Especially the embryoid body has the capacity to produce the three embryonic germ layers.

In an embodiment of the invention, step a) lapses at least 1 days, and especially 2 days.

In particular, the said inducing neuronal differentiation culture medium comprises ROCK inhibitor and/or a low fibroblast growth factor-basic concentration. Particularly, the fibroblast growth factor-basic concentration is at most 5 ng/ml, especially 4 ng/ml.

The 3D embedding system may be for example a mixed floating gel composed of matrix protein such as Matrigel and/or Collagen I.

Step b) may comprises a sub-step of disposing the 3D embedding system comprising the embryoid body into a spinning bioreactor for increasing 3D differentiation.

Advantageously, step c) does not comprise mechanical manipulation of the growing tissue, diminishing the risk of development impairment.

In one embodiment of the invention, step lapses at least 30 days, especially at least 60 days, notably at least 100 days, in particular at least 150 days, for example 200 days.

The human pluripotent stem cell wherein CHD1L gene is knocked out or knocked down, may be obtained by any known method in the art for this purpose, and with the ones above described.

The human pluripotent stem cell wherein CHD1L protein's activity is impaired may be obtained by any method known in the art, and especially the ones above described.

The invention also relates to a human pluripotent stem cell-derived, in vitro generated, retinal tissue characterized in that CHD1L activity is decreased obtained by the method for obtaining a retinal tissue as above defined.

The different embodiments related to the retinal tissue and the ones related to the method for obtaining a retinal tissue apply *mutatis mutandis* to this object of the invention.

The invention also relates to a method for obtaining a retinal cell line by culturing retinal progenitors cells isolated from the retinal tissue of the invention.

Especially, the said method comprises the following steps:
a) obtaining a retinal tissue with the method as above defined,
b) isolating retinal progenitor cells from the retinal tissue, especially by use of flow cytometry or magnetic bead sorting technology,
c) culturing the isolated retinal progenitor cells in a culture medium comprising one or more differentiation factors to differentiate the retinal progenitors cells into retinal cells of the retinal cell line,
d) isolating the retinal cells, especially by use of flow cytometry or magnetic bead sorting technology,
e) culturing the said isolated retinal cells in a specific culture medium containing growth factors, anti-apoptotic factors, extracellular matrix proteins, and
f) recovering the retinal cell line.

### Method for assessing the efficiency of a drug candidate

The invention also relates to a method for assessing the efficiency of a drug candidate for treating a retinal disease comprising:
a) producing a retinal tissue with the method as above defined or a retinal cell line with the method as above defined, optionally wherein the retinal tissue or the retinal cell line has a genetic mutation or other impairment,
b) administering the drug candidate to the retinal tissue or the retinal cell line, and
c) determining the effect of the drug candidate onto the retinal tissue or the retinal cell line.

In an embodiment of the invention, during step b), the drug candidate is administered once or at least twice.

In an embodiment of the invention, the drug candidate comprises a protein, a virus, a RNA molecule, a DNA molecule, a gene therapy, a small molecule, a gene editor, a base editor, an RNA editor, a small molecule targeting DNA/RNA, a cell therapy, a genome or base editing technology, an antibody, a nanobody or a nanoparticle.

In an embodiment of the invention, during step c), the drug candidate's therapeutic effect on the retinal tissue or the retinal cell line is monitored overtime, especially with episodic live imaging.

The effect of the drug during step c) may be determined by any modification in the cells of the retinal tissue or retinal cell line morphology and/or composition, including but not limited to expression of genes quantification proteins translation amount quantification and electrophysiology parameters.

The present invention also envisages that the retinal tissue, or a cell derived from said retinal tissue, can be used in a drug discovery screen; toxicity assay; research of tissue embryology, cell lineages, and differentiation pathways; gene expression studies including recombinant gene expression or gene expression, such as using an inducible Cre-based expression of cDNAs or CRISPR components 3' to a Lox-Stop-Stop-Lox element and 5' to a 2A peptide linked fluorescent protein marker; research of mechanisms involved in tissue injury and repair; research of inflammatory and infectious diseases; studies of pathogenetic mechanisms; or studies of mechanisms of cell transformation and aetiology of retinal disease.

### Method of treatment

The invention also relates to the retinal tissue as above defined or the retinal cell line as above defined for use in the treatment of a retinal disease or a retinal disorder in a subject.

Accordingly, the retinal tissue of the invention or the retinal cell line as above defined is used as a therapeutic agent and administered in an effective amount to the subject.

As used herein, the term "subject" may be used interchangeably with the term "patient" or "individual" and may include an "animal" and in particular a "mammal." Mammalian subjects may include humans and other primates, domestic animals, farm animals, and companion animals such as dogs, cats, guinea pigs, rabbits, rats, mice, horses, cattle, cows, and the like. Said subject may have been previously diagnosed with a retinal disease or a retinal disorder.

The term "disorder" or "disease" can be used interchangeably and refers to changes in cells, tissues, organs or organisms as compared to normal (healthy) cells, tissues, organs or organisms. In some instances, the physiological functions related to natural organ function, homeostasis, aging, or regeneration may be changed, like abnormal organ development, inflammatory diseases, autoimmune diseases, chronic diseases, infectious disease or in cancer.

As used herein, the term "retinal disorder" or "retinal disease" refers generally to a disorder of the retina. In one embodiment, the retinal disorder or retinal disease is associated with oxidative stress, decreased viability, for example, death, of cone cells, and/or rod cells. Moreover, in a particular embodiment, a retinal disorder or retinal disease is associated with blood vessel leakage and/or growth, for example, as this is the case with diabetic retinopathy. In another embodiment, instead, the retinal disorder or disease is characterized primarily by reduced viability of cone cells and/or rod cells. In certain embodiments, the retinal disorder or retinal disease is a genetic disorder. In another embodiment, the retinal disorder or retinal disorder is age-related macular degeneration. In another embodiment, the retinal disorder is cone-rod dystrophy. In another embodiment, the retinal disorder is rod-cone dystrophy. In certain embodiments, the retinal disorder is not associated with diabetes and/or diabetic retinopathy. In further embodiments, the retinal disorder or disease is not NARP (neuropathy, ataxia, and retinitis pigmentosa). In yet further embodiments, the retinal disorder or disease is not a neurological disorder. In certain embodiments, the retinal disorder or disease is not associated with a compromised optic nerve and/or disorders of the brain. In the foregoing embodiments, the retinal disorder or disease is associated with a compromised photoreceptor cell, and is not a neurological disorder.

In an embodiment of the invention, the retinal disease or retinal disorder is selected in the group consisting of inherited retinal dystrophy, diabetic retinopathy, retinopathy of prematurity, macular degeneration, age-related macular degeneration, Usher syndrome, Stargardt disease, Retinitis Pigmentosan, Bardet-Biedl syndrome, Senior-Loken syndrome, Leber congenital amaurosis, Joubert syndrome and Meckel syndrome.

The terms "treatment", "treating" and the like are used herein to generally mean obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment" as used herein covers any treatment of a disease in a mammal, and includes: (a) preventing the disease from occurring in a subject which may be susceptible to the disease but has not yet been diagnosed as having it; (b) inhibiting the disease, *i.e.,* arresting its development; or (c) relieving the disease, *i.e.,* causing regression of the disease. The therapeutic agent may be administered before, during or after the onset of disease or injury. The treatment of ongoing disease, where the treatment stabilizes or reduces the undesirable clinical symptoms of the patient, is of particular interest. The subject therapy will desirably be administered during the symptomatic stage of the disease, and in some cases after the symptomatic stage of the disease.

An "effective amount" or "effective dose" as used herein is an amount which provides the desired effect. For therapeutic purposes, an effective amount is an amount sufficient to provide a beneficial or desired clinical result. The preferred effective amount for a given application can be easily determined by the skilled person taking into consideration, for example, the size, age, weight of the subject, the type of disease/disorder to be prevented or treated, and the amount of time since the disease/disorder began. In the context of the present invention, in terms of prevention or treatment, an effective amount is an amount that is sufficient to achieve a desired therapeutic and/or prophylactic effect, e.g., an amount which results in the prevention of, or a decrease in, the symptoms associated with a retinal disease or a retinal disorder.

In one embodiment of the invention, the treatment is cell replacement therapy.

Preferably, the cell replacement therapy uses target retinal cells for treating the retinal disease or retinal disorder at a retinal area of the subject.

Especially the cell replacement therapy may comprise the following steps:
a) collecting cells from the subject or from an healthy donor,
b) producing a retinal tissue or a retinal cell line with the method of production as above defined,
c) collecting or isolating the target retinal cells, especially by flow cytometry or magnetic bead sorting technology,
d) optionally culturing the target retinal cells in a specific culture medium containing growth factors, anti-apoptotic factors, and extracellular matrix proteins, especially in case the target retinal cells are isolated from the retinal tissue at step c),
e) carrying out minimally invasive surgical techniques to transplant the target retinal cells into the subject's retinal area, and
f) optionally after transplantation, providing the subject with immunosuppressive therapy and retinal protection measures to promote the survival and functional recovery of transplanted target retinal cells.

In an embodiment of the invention, the target retinal cells are selected in the group consisting of retinal progenitors cells, cycling retinal progenitor cells, retinal ganglionic cells, amacrine/horizontal cells, photoreceptors/cones and mixture thereof.

### Pharmaceutical composition

The invention finally relates to a pharmaceutical composition for treating a retinal disease or retinal disorder comprising retinal cells isolated from the retinal tissue as above defined or from the retinal cell line as above defined and a pharmaceutically acceptable vehicle.

In one embodiment of the invention, the retinal cells comprise cells selected in the group consisting of retinal progenitors cells, cycling retinal progenitor cells, retinal ganglionic cells, amacrine/horizontal cells and photoreceptors/cones.

The term "pharmaceutical vehicle" as used herein refers to a carrier or inert medium used as solvent or diluent in which the pharmaceutically active agent is formulated and/or administered. Non-limiting examples of pharmaceutically acceptable vehicles include dispersants, solubilisers, stabilisers, preservatives, etc. Pharmaceutically acceptable vehicles that can be used in formulations (liquid and/or injectable and/or solid) include methylcellulose, phydroxymethylcellulose, carboxymethylcellulose, cyclodextrins, polysorbate 80, mannitol, gelatine, lactose, vegetable or animal oils, acacia, etc.

The said pharmaceutical composition may be in the form of a physiological, isotonic and buffered saline solution compatible with pharmaceutical use and known to the person skilled in the art. Said pharmaceutical composition may be formulated in any pharmaceutically acceptable form, such as for example in the form of an injectable suspension, gels, oils, tablets, suppositories, capsules, etc., possibly used by means of galenic forms or devices ensuring prolonged and/or delayed release. For this type of formulation, an agent such as cellulose, carbonates or starches is advantageously used.

The retinal cells within the pharmaceutical composition according to the present invention may be used alone or in combination with at least one other therapeutically active compound, such as, for example, an anticancer compound. The use of the said pharmaceutical composition and the said therapeutically active compound may be simultaneous, separate or spread over time, in particular during treatment of a subject suffering from cancer.

### BRIEF DESCRIPTION OF THE DRAWINGS

Other aspects and advantages of the present invention will become apparent from the following examples, which are to be considered illustrative and non-limiting, and from the attached figures in which:
**Figure 1** represents pictures of 60 days *in vitro* (DIV) human organoid derived from *CHD1L*^{+/+} (A) and the two *CHD1L*^{-/-} mutant Line 1 (B). Scale bar, 1 mm.
**Figure 2** represents immunostaining against TUJ1 (neuronal cytoskeleton marker, dark grey) and SOX2 (neural progenitor cells, light grey) on 52 DIV human organoid either *CHD1L*^{-/-} *(B:* mutant Line 1) or control (A).
**Figure 3** represents semi-quantitative analysis of TUJ1 protein expression in *CHD1L*^{*-*/}*⁻ (B:* mutant Line 1) and control (A) human organoid normalized with β-Tubulin. Data shown as mean ± SEM of 8 human organoid per condition; representative of N=2 batches of generated organoids; Student's t-test.
**Figure 4** represents nearest neighbour graph Uniform Manifold Approximation and Projection (wnnUMAP) of control (hollow circle) and *CHD1L*^{*-*/*-*} mutant Line 1 (solid square) multiome analysis combined and integrated on the same graph. N=12 organoids, 4,019 cells.
**Figure 5** represents a Sankey diagram of the proportion of nuclei positive for either the ventral telencephalic marker *FOXG1* (C: FoxG1 positive), or the eye territory marker *SIX3* (normalized expression > 1; D: SIX3 positive) or none of them (E: FOXG1 negative and SIX3 negative) for control (A) and *CHD1L*^{*-*/*-*} (B) human organoid. Percentage of nuclei are indicated for each category.
**Figure 6** represents *t*-SNE dimensionality reduction of 4,019 cells based on gene and target region enrichment scores of eRegulons. Cells are coloured according to their genotypes *CHD1L*^{*+*/*+*} (control, hollow circle) and *CHD1L*^{*-*/*-*} mutant Line 1 (solid square) and were analysed for gene expression and chromatin accessibility in 60 days *in vitro* human organoid.

### EXAMPLES

Self-organizing cerebral organoids grown from pluripotent stem cells combined with single-cell genomic technologies provide opportunities to examine gene regulatory networks underlying human brain development and diseases. To better characterize the role of CHD1L during cerebral development and to test the possibility that CHD1L plays a role during brain regionalization, the inventors derived CHD1L+/+ and CHD1L -/- hiPSC (mutant Line 1) into cerebral organoids for 60 days *in vitro* (DIV). The inventors chose to build organoids through a self-organization process by providing a permissive environment with minimal external cues which allowed them to determine the intrinsic capacity of the control and CHD1L mutant hiPSC to undergo *in vivo-like* morphogenesis.

### Material and Method

### hiPSC culture

Control human induced pluripotent stem cells (hiPSC GM8330-8), derived from adult fibroblasts, were kindly provided by Prof. M.E. Talkowski. The cells were maintained on Matrigel-coated dish (Corning) with mTESR^{™} (StemCell) and incubated at 37 °C in a humidified atmosphere with 5% CO₂. The inventors transfected the human iPSCs with the pSpCas9(BB)-2A-GFP gRNA plasmid using Lipofectamine^{™} Stem Reagent, adapting the protocol described in Tai, D.J.C., et al. (2016), Engineering microdeletions and microduplications by targeting segmental duplications with CRISPR. Nat. Neurosci. 19, 517-522. At 48 hours post-transfection, the hiPSCs were dissociated into a single cell suspension with Accutase and resuspended in PBS with 10 µM ROCK inhibitor (Santa Cruz^{®}). All samples were filtered through 5mL polystyrene tubes with 35 µm mesh cell strainer caps (BD Falcon^{®}, 352235) immediately before being sorted. After adding the viability dye DAPI (BD Bioscience^{®}), single GFP+ DAPI- cells were isolated by fluorescence-activated cell sorting (FACS) gated for a high level of GFP expression and sorted, with one cell placed into each well of Matrigel-coated 96-well plates by BD FACS Aria II^{®} with 100-mm nozzle under sterile conditions. The medium was supplemented with CloneR^{™} (StemCell) from Day 0 to Day 4 according to the manufacturer's instructions.

### hiPSC-editing guide RNA design and preparation

The inventors used the CRISPR MIT tool (http://crispr.mit.edu) to generate a guide RNA targeting the exon 1 of CHD1L (5'-TCATACTGAGGGCCGAGCCGAGG-3', chr1: 147242763-147242785, GRCh38). The gRNA was cloned into pSpCas9(BB)-2A-GFP (Addgene, PX458) plasmid. Validation of the guide sequence in the gRNA vector was confirmed by Sanger Sequencing^{®}. Before transfection, all plasmids were purified from PureLink^{™} HiPure Plasmid Midiprep Kit according to the manufacturer's instruction (Thermo Fisher Scientific^{®}).

### Colony screening and western blot validation

Genomic DNA from two thirds of each hiPSC colony (obtained around 14 days after sorting) were extracted by using Quick-DNA 96 kit (Zymo research^{®}) and screened by PCR (using the indicated primers: forward 5'- GGAAGTTGGGAGGGAGGT-3' and reverse 5' GCTGATCTCACCACGTTTCC-3') followed by Sanger Sequencing^{®}. hiPSC screening validation: 100 µg of total iPSC protein lysate was prepared in RIPA buffer and Protease Inhibitor Cocktail from control and two CHD1L-edited iPSCs lines (mutant Line 1), diluted 1× final with Laemmli buffer and DTT 0.1 M, boiled for 5 min and then separated by SDS-PAGE on 10% polyacrylamide gels. Resolved proteins were transferred to nitrocellulose membranes and blocked in 3% milk 1× TBS for 1 h at room temperature prior to incubation with either anti-CHD1L (2170C3a) antibody (Santa Cruz^{®}, sc-81065, 1:200) or anti-β-Tubulin (1:10,000, produced in house). Membranes were washed and incubated in goat anti-mouse peroxidase secondary antibody (Jackson Immuno Research^{®}, 1:10,000). Blots were developed using Immobilion Western (Millipore^{®}, France) according to the manufacturer's instructions.

### ATAC-seq library preparation

Assay for transposase accessible chromatin was performed on hiPSC-derived hNPC using a derived protocol from Buenrostro, J.D., Wu, B., Chang, H.Y., and Greenleaf, W.J. (2015), ATAC-seq: A Method for Assaying Chromatin Accessibility Genome-Wide. Curr. Protoc. Mol. Biol. 109, 21.29.1-21.29.9. A total of 50,000 cells from GM8330-8 (control line CHD1L+/+), mutant Line 1 and mutant Line2 (isogenic CHD1L mutant lines) lines were resuspended in resuspension buffer (10mM Tris-HCl pH 7.5, 10mM NaCl, 3mM MgCl2) and then lysed in lysis buffer (Resuspension buffer + 0.1% NP40 + 0.1% tween-20+ 0.01% Digitonin) and incubated 3 min on ice. Then, 1 mL of wash buffer was added (resuspension buffer + 0.1% Tween-20). Cells were centrifugated 10 min at 500G at 4°C and supernatant was discarded. Transposition reaction mix (Illumina^{®}, Tagment DNA Enzyme and Buffer Small Kit, 20034197) was added to pellet and incubated 30 min at 37°C in a thermomixer at 1,000 rpm. DNA fragments were isolated using Qiagen^{®} MinElut Reaction Cleanup kit. DNA fragments were amplificated and libraries were generated by PCR using appropriate primers as described in Buenrostro *et al.* and NEBNext High-Fidelity 2X PCR Master mix (NEB, M0541S). Finally, libraries were purified using SRIselect beads (Beckman Coulter) by a one-sided purification to remove primers. Sequencing was performed using Illumina^{®} HiSeq 4000 with 100 bp paired-end sequencing.

### ATAC-seq bioinformatic analyses

Data analysis was performed using the Encode ATAC-seq pipeline (v1.4.2). Adapter sequences were removed and low-quality ends were trimmed. Reads were mapped onto the hg38 assembly of Homo Sapiens genome using Bowtie2 (v2.2.6) choosing the zero multi-mapping option. Mitochondrial reads were removed. The Peak calling was performed using MACS2 (v2.1.1.20160309). Finally, the optimal overlap peaks were used for downstream analyses. Peaks from different conditions were merged to form a consensus peak set. The peaks were annotated using annotatePeaks.pl script in Homer program and with Ensembl 98 database. The read coverage for each sample was calculated with multicov function from bedtools program (v2.26.0). Differential analyses of Control GM8330-8 vs Line 1 isogenic mutants were performed using the Bioconductor package DESeq2 (v1.16.1)132. For the TOBIAS analysis (Transcription factor occupancy prediction by investigation of ATAC-seq signal) of enriched motif elements, the pipeline snakemake (v0.12.11) was used62. ATACseq datasets were compared to H3K4me2 peaks from CUT&RUN analysis performed in this study using seqMINER. Representative traces were generated using Figeno.

### Results

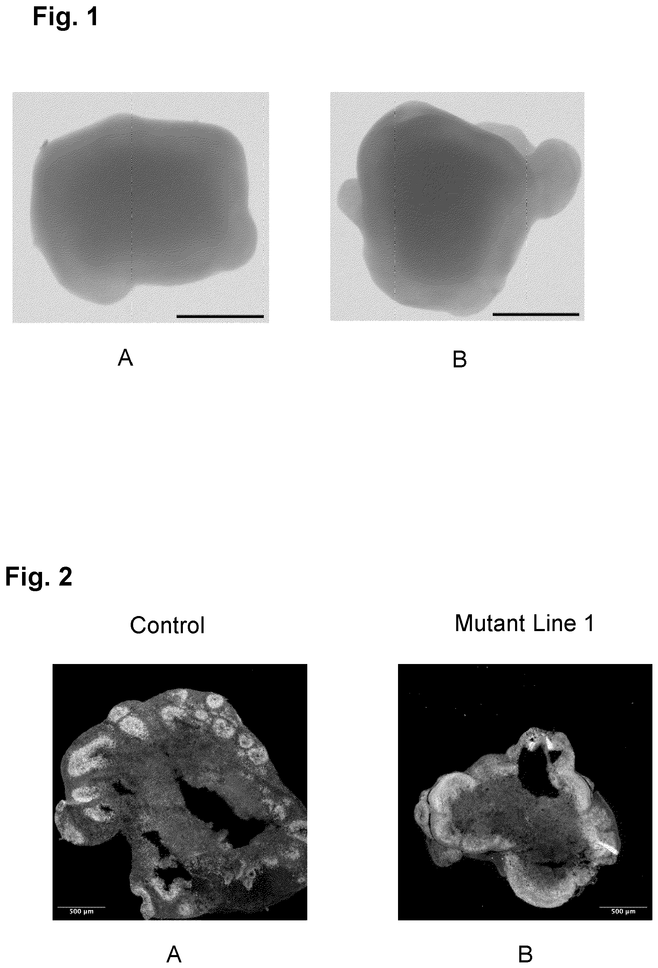

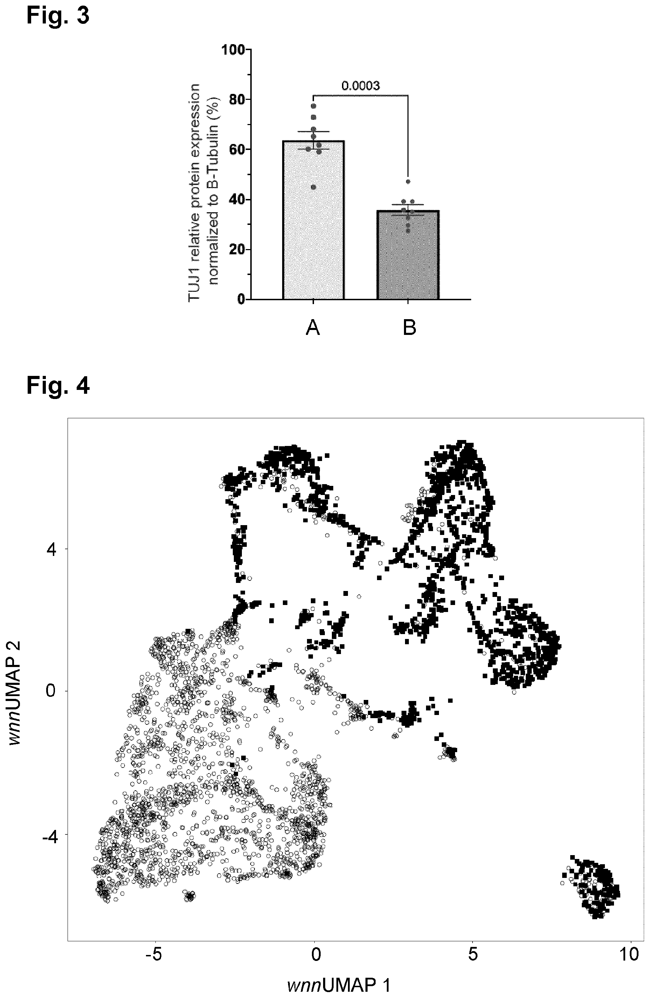

While CHD1L-/- mutant Line 1 exhibited apparent similar morphology as CHD1L+/+ control organoids during the *in vitro* maturations, (Figure 1). At 52 DIV, control and mutant organoids were positive for both SOX2 (neural progenitor cells marker) and TUJ1 (neural cytoskeleton marker) (Figure 2). The inventors noted typical structures of cerebral organoids including rosettes (SOX2+) and cortical plate layers (TUJ1+). However, the inventors observed a decreased level of TUJ1 protein expression in CHD1L-/- mutant organoids compared to control organoids at 60 DIV (Figure 3), suggesting impaired neurogenesis in organoids lacking CHD1L.

To determine which cell populations are the most affected by the absence of CHD1L, the inventors employed single nuclei Gene expression and ATAC-seq multiome (referred as snMultiome) analysis to simultaneously profile the transcriptional and chromatin states of control and CHD1L mutant Line 1 60 DIV organoids. A total of 4,019 cells were individually sequenced, including 2,385 cells extracted from control organoids, and 1,634 cells from CHD1 L-/- mutant organoids mutant Line 1(Figure 4). To their surprise, the inventors observed almost no overlap of the cell clusters from control and CHD1L-/- mutant organoids suggesting a dramatic effect of the absence of CHD1L on cell identity (Figures 4).

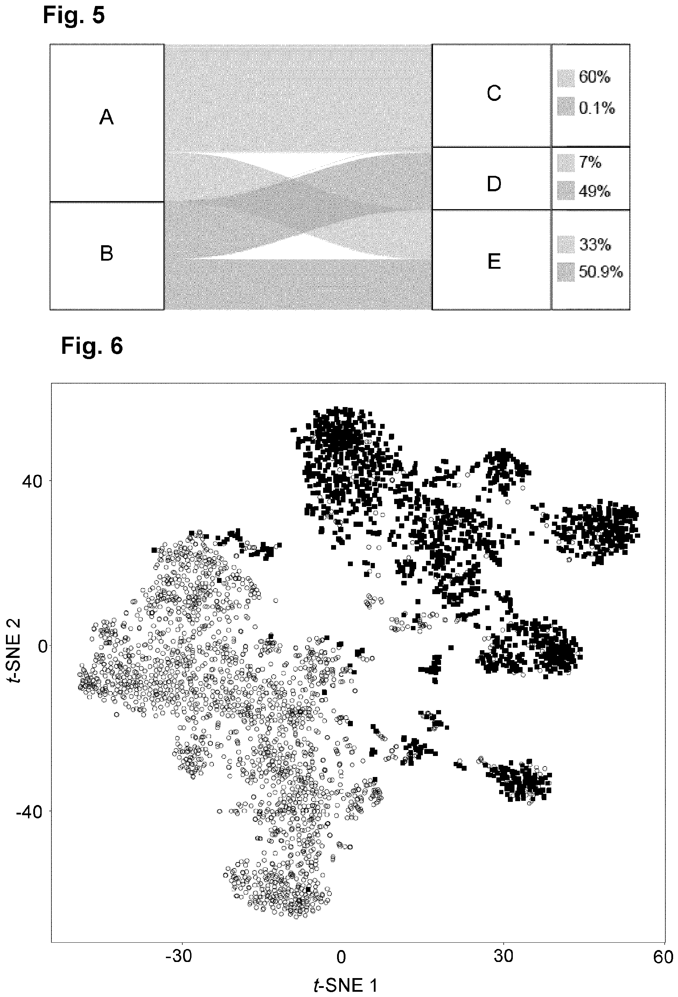

The Seurat weighted nearest neighbour method was used to compute a neighbour graph which was visualized with Uniform Manifold Approximation and Projection (UMAP). A total of 16 clusters were annotated based on expression of marker genes (Figure 5). The inventors found groups with telencephalic identity (FOXG1, PAX6, SOX2) including radial glial cells, cycling radial glial cells, intermediate progenitor cells (IPC), cycling IPC, and two groups of neurons including immature and excitatory neurons. The inventors also identified groups with retinal identity (SIX3, RORB, VSX2, OTX2) including Retinal Progenitor Cells, Cycling Retinal Progenitor Cells, Retinal Ganglionic Cells, Amacrine/Horizontal cells and Photoreceptors/Cones. The inventors further found cell clusters from Forebrain Telencephalic/Diencephalic boundary, Mesenchyme, Hindbrain and Choroid Plexus and presented in the below Table 1.

**Table 1**

| **Cell type** | **Control** | **CHD1L mutant Line 1** |
|---|---|---|
| Amacrine/Horizontal Cells | 8 | 120 |
| Choroid Plexus | 58 | 136 |
| Cycling Radial GC | 45 | 0 |
| Cycling Retinal PC | 3 | 68 |
| Excitatory Neurons | 701 | 0 |
| Hindbrain | 6 | 45 |
| Immature neurons | 625 | 3 |
| Intermediate Progenitor Cells | 154 | 3 |
| Mesenchymal Cells | 26 | 16 |
| Photoreceptors/cones | 38 | 332 |
| Radial Glial Cells | 381 | 1 |
| Retinal Ganglionic Cells | 81 | 314 |
| Retinal Progenitor Cells | 54 | 361 |
| Telencephalon/Diencephalon boundary | 182 | 101 |
| Transient Retinal Precursor Cells | 16 | 97 |
| Uncertain | 7 | 37 |

As shown in Table 1, the expression profiles of the control and CHD1L Mutant Line 1 are almost exclusive for each other, demonstrating the critical role of the absence of CHD1 L expression for retinal fate.

Strikingly, the inventors observed that among the 2,385 sequenced nuclei from CHD1L+/+ cerebral organoids, a total of 1,436 nuclei were FOXG1-positive (60.2%) whereas 169 nuclei were SIX3-positive (7.1%). To the contrary, the inventors found only three FOXG1-positive nuclei (0.2%) among the 1,634 nuclei and a total of 809 SIX3-positive nuclei (49.5%) for CHD1L-/- (Line 1) cerebral organoids (Figure 5).

These findings exemplified the profound cell fate difference between control and CHD1L mutant organoids, the latter expressing retinal-specific genes and resembling to mature hiPSC-derived retinal organoids at 60 DIV.

The inventors finally performed SCENIC+ workflow to catalogue the set of enhancer-driven regulons that form gene regulatory networks (GRNs) in both wildtype and CHD1L mutant organoids (Figure 6). SCENIC+ identified 55 activator and 12 repressor eRegulons. SCENIC+ recovered well-known master regulators of excitatory neurons (NEUROD2, MEF2C, TBR1 and FOXG1), cycling and non-cycling radial glial cells (EOMES and SIX3, RORB, ONECUT1 respectively), retinal ganglionic cells (RAX, CRX, RXRG, NEUROD1), transient retinal precursor cells (RFX2, MITF) and photoreceptors/cones (ISL1, POU2F2, EBF1/3). The majority of the top five cell-type-specific transcription factors showed co-binding to shared enhancers. Individual eRegulon visualization further confirmed the presence of transcription factors favouriting telencephalic fate (FOXG1(+), SOX2(+), NEUROD6(+)) and the subsequent positive regulation of their associated genes and regions in CHD1L+/+ organoids whereas transcription factors associated with retinal fate specification (OTX2(+), VSX2(+), SIX3(+)) were found in CHD1L mutant organoids.

Taken together, these data revealed that CHD1L acts as a master regulator of forebrain cell-fate decision and promotes telencephalon fate during brain regionalization. Consequently, absence of CHD1L appears as a key factor for directing pluripotent stem cell to retinal fate.

## Claims

1. A human pluripotent stem cell-derived, *in vitro* generated, retinal tissue **characterized in that** CHD1L activity is decreased.

2. The retinal tissue according to claim 1, which is a three-dimensional retinal organoid.

3. The retinal tissue according to claim 1 or 2, wherein the human pluripotent stem cell is a human embryonic stem cell or a human induced pluripotent stem cell.

4. The retinal tissue according to any of claims 1 to 3, wherein the proportion of cells of the retinal tissue positives for FOXG1 is less than 1% and/or the proportion of cells positives for SIX3 is more than 40%.

5. The retinal tissue according to any of claims 1 to 4, wherein it comprises at least one group of retinal cells selected in the group consisting of retinal progenitors cells, cycling retinal progenitor cells, retinal ganglionic cells, amacrine/horizontal cells, photoreceptors/cones, Rods photoreceptors, bipolar cells and glia müller-type cells.

6. A retinal cell line derived from the retinal tissue according to any of claims 1 to 6.

7. The retinal cell line according to claim 7, which is selected in the group consisting of a retinal progenitor cell line, a cycling retinal progenitor cell line, a retinal ganglionic cell line, an amacrine/horizontal cell line, a photoreceptors/cones cell line, Rods photoreceptors, bipolar cells and glia müller-type cells.

8. A method for obtaining a retinal tissue according to any of claims 1 to 6 comprising culturing human pluripotent stem cell wherein CHD1L activity is decreased to form a retinal tissue.

9. The method of claim 8, comprising method comprises the following steps:
a) culturing the human pluripotent stem cells wherein CHD1L activity is decreased in an inducing neuronal differentiation culture medium until obtaining an embryoid body,
b) recovering the embryoid body and placing it into a 3D embedding system,
c) culturing the embryoid body within the 3D embedding system until obtaining a retinal tissue.

10. A method for obtaining a retinal cell line by culturing a retinal progenitor cell isolated from the retinal tissue according to any of claims 1 to 6.

11. A method for assessing the efficiency of a drug candidate for treating a retinal disease or retinal disorder comprising:
a) producing a retinal tissue with the method according to claim 9 or a retinal cell line with the method according to claim 10, optionally wherein the retinal tissue or the retinal cell line has a genetic mutation or other impairment,
b) administering the drug candidate to the retinal tissue or the retinal cell line, and
c) determining the effect of the drug candidate onto the retinal tissue or the retinal cell line.

12. The retinal tissue according to any of claims 1 to 6 or the retinal cell line according to claim 7 or 8 for use in the treatment of a retinal disease or a retinal disorder in a subject.

13. The retinal tissue according to any of claims 1 to 6 or the retinal cell line according to claim 7 or 8 for use according to claim 12, wherein the retinal disease is selected in the group consisting of inherited retinal dystrophy, diabetic retinopathy, retinopathy of prematurity, macular degeneration, age-related macular degeneration, Usher syndrome, Stargardt disease, Retinitis Pigmentosan, Bardet-Biedl syndrome, Senior-Loken syndrome, Leber congenital amaurosis, Joubert syndrome and Meckel syndrome.

14. The retinal tissue according to any of claims 1 to 6 or the retinal cell line according to claim 7 or 8 for use according to claim 11 or 12, wherein the treatment is cell replacement therapy.

15. A pharmaceutical composition for treating a retinal disease or retinal disorder comprising retinal cells isolated from the retinal tissue according to any of claims 1 to 6 or from the retinal cell line according to claim 7 or 8 and a pharmaceutically acceptable vehicle.
